# EUROPÄISCHE PATENTSCHRIFT

(11) **EP 1 837 926 B1**
(45) Veröffentlichungstag und Bekanntmachung des Hinweises auf die Patenterteilung: **07.05.2008**
(21) Anmeldenummer: 06005687.6
(22) Anmeldetag: 21.03.2006
(51) Int. Cl.: H01L 51/00, C07D 233/24, C07D 235/02, C07D 235/20, C07D 277/66

(54) **Heterocyclisches Radikal oder Diradikal, deren Dimere, Oligomere, Polymere, Dispiroverbindungen und Polycyclen, deren Verwendung, organisches halbleitendes Material sowie elektronisches Bauelement**
Heterocyclic radicals or diradicals and their dimers, oligomers, polymers, di-spiro and polycyclic derivatives as well as their use in organic semiconductor materials and electronic devices.
Radicaux ou diradicaux hétérocycliques, leur dimères, oligomères, polymères, composés spiro et polycycliques. Leur usage dans des semi-conducteurs organiques et dispositifs électroniques.

(43) Veröffentlichungstag der Anmeldung: 26.09.2007
(73) Patentinhaber: Novaled AG, 01307 Dresden (DE)
(72) Erfinder: Limmert, Michael, 01277 Dresden (DE); Hartmann, Horst, 01326 Dresden (DE); Zeika, Olaf, 01187 Dresden (DE); Werner, Ansgar, 01277 Dresden (DE); Ammann, Martin, 01159 Dresden (DE)
(74) Vertreter: Bittner, Thomas L.

(56) Entgegenhaltungen:
- US-A1- 2005 040 390
- A. R. SIEDLE AND R. B. JOHANNESEN: "Reduction of the 1,3-dithiolium cation with hexacarbonylvanadate" JOURNAL OF ORGANIC CHEMISTRY, Bd. 40, Nr. 13, 1975, Seite 2202, XP002396000
- H. JADAMUS, Q. FERNANDO AND H. FREISER: "Metal-ion induced rearrangements of bisbenzthiazolines to Schiff-base chelates" JOURNAL OF THE AMERICAN CHEMICAL SOCIETY, Bd. 86, 1964, Seiten 3056-3059, XP002396001
- E. J. COREY, F. A. CAREY AND R. A. WINTER: "Stereospecfic syntheses of olefins from 1,2-thionocarbonates and 1,2-trithiocarbonates. Trans-cycloheptene" JOURNAL OF THE AMERICAN CHEMICAL SOCIETY, Bd. 87, Nr. 4, 1965, Seiten 934-935, XP002396002
- E. BAYER AND E. BREITMAIER: "Die reaktion von Benzil mit 2-Aminothiophenol" TETRAHEDRON LETTERS, Bd. 15, 1966, Seiten 1689-1693, XP002396003
- H. G. MAUTNER: "Potential deoxyribonucleic acid cross-linking agents. 8,8'-bispurines." JOURNAL OF ORGANIC CHEMISTRY, Bd. 26, 1961, Seiten 1914-1917, XP002396004
- R. C. ELDERFIELD AND E. C. MCCLENACHAN: "Pyrolisis of the products of the reaction of o-aminobenzenethiols with ketones" JOURNAL OF THE AMERICAN CHEMICAL SOCIETY, Bd. 82, 1960, Seiten 1982-1988, XP002396005
- M. G. MILES, J. S. WAGER AND J. D. WILSON: "Reactions of 4,5-dicyano-1,3-dithiole-2-thione and 1,3-dithiol-2-one with tervalent phosphorous compounds" JOURNAL OF ORGANIC CHEMISTRY, Bd. 40, Nr. 18, 1975, Seiten 2577-2582, XP002396006

## Beschreibung

Die vorliegende Erfindung betrifft heterocyclische Radikale oder Diradikale, deren Dimere, Oligomere, Polymere, Dispiroverbindungen und Polycyclen, deren Verwendung, organische halbleitende Materialien sowie elektronische Bauelemente.

Es ist bekannt, organische Halbleiter durch Dotierung hinsichtlich ihrer elektrischen Eigenschaften, insbesondere ihrer elektrischen Leitfähigkeit, zu verändern, wie dies auch bei anorganischen Halbleitern, wie Siliciumhalbleitern, der Fall ist. Hierbei wird durch Erzeugung von Ladungsträgern im Matrixmaterial eine Erhöhung der zunächst recht niedrigen Leitfähigkeit sowie je nach Art des verwendeten Dotanden eine Veränderung im Fermi-Niveau des Halbleiters erreicht. Eine Dotierung führt hierbei zu einer Erhöhung der Leitfähigkeit von Ladungstransportschichten, wodurch ohmsche Verluste verringert werden, und zu einem verbesserten Übergang der Ladungsträger zwischen Kontakten und organischer Schicht.

Die bisher verwendeten anorganischen Dotanden wie Alkali- oder Erdalkalimetalle (z.B. Cäsium) oder Lewis-Säuren (z.B. FeCl₃) sind bei organischen Matrixmaterialien aufgrund ihrer hohen Diifusionskoeffizienten meist nachteilig, da die Funktion und Stabilität der elektronischen Bauelemente beeinträchtigt wird. Mit diesen anorganischen Dotanden sind weiterhin Schwierigkeiten in der Produktion verbunden, da sie in der Regel einen hohen Dampfdruck bei Raumtemperatur aufweisen und in Vakuumprozessen die Produktionsanlagen kontaminieren können. Insbesondere Alkali- und Erdalkalimetalle haben den weiteren Nachteil, dass ihre Verwendung durch ihre starke Reaktivität mit Luft erschwert ist. Ferner ist es bekannt, Dotanden über chemische Reaktionen in dem halbleitenden Matrixmaterial freizusetzen, um Dotanden bereitzustellen. Das Oxidationspotential der derart freigesetzten Dotanden ist jedoch für verschiedene Anwendungsfälle, wie insbesondere für organische Leuchtdioden (OLED), oftmals nicht ausreichend. Ferner werden bei Freisetzung der Dotanden auch weitere Verbindungen und/oder Atome, beispielsweise atomarer Wasserstoff, erzeugt, wodurch die Eigenschaften der dotierten Schicht bzw. des korrespondierenden elektronischen Bauelements beeinträchtigt werden.

Ferner haben als Dotanden verwendete Verbindungen oftmals kein ausreichend niedriges lonisierungspotential für den jeweiligen Anwendungsfall.

US 2005/04 03 90 offenbart heterocyclische Verbindungen als Dotand zur Dotierung eines organischen halbleitenden Materials.

Der vorliegenden Erfindung liegt die Aufgabe zugrunde, neue Verbindungen bereitzustellen, die als n-Dotanden, als Injektionsschicht oder als Blockerschicht eingesetzt werden können, wobei die Verbindungen auch zur Herstellung von Elektronentransportmaterialien für organische Leuchtdioden ausreichend niedrige Oxidationspotentiale aufweisen, ohne störende Einflüsse auf das Matrixmaterial sein und eine wirksame Erhöhung der Ladungsträgeranzahl im Matrixmaterial bereitstellen sollen und vergleichsweise einfach handhabbar sind.

Weitere Aufgaben der vorliegenden Erfindung liegen in der Angabe möglicher Verwendungen dieser Verbindungen, in der Bereitstellung organischen halbleitenden Materials und eines elektronischen Bauelements oder optoelektronischen Bauelementes, in denen die Verbindungen z.B. in photoinitiierten Speichern eingesetzt werden können.

Die erste Aufgabe wird durch die heterocyclischen Radikale oder Diradikale, deren Dimere, Oligomere, Polymere, Dispiroverbindungen und Polycyclen gelöst, die in Anspruch 1 aufgeführt sind. Die weiteren Aufgaben werden durch die Gegenstände der weiteren unabhängigen Ansprüche gelöst, während bevorzugte Ausführungsformen in den Unteransprüchen wiedergegeben sind.

Die Reste R₀₋₁₉, R₂₁, R₂₂ und R₂₃ können bevorzugt substituiertes Aryl sein, wobei die Substituenten vorzugsweise elektronenschiebende Reste sind, beispielsweise Dialkylamin, Julolidyl, Diarylamin, Alkylarylamino, Diheterocyclylamin, Diheteroalkylamin, Alkoxy, Aryloxy, Alkylmercaptyl, Arylmercaptyl, Alkyl, Sililyl, Halogenalkyl, Cycloalkyl, Halogencycloalkyl, Alkenyl, Alkinyl, Trialkylsililylalkinyl oder Styryl.

Alle beanpruchten Verbindungen können perfluoriert oder teilfluoriert vorliegen, insbesondere gesättigte Brücken und Henkel.

Überraschenderweise wurde festgestellt, daß ein wesentlich stärkerer und/oder stabilerer Dotand als bei bisher bekannten Donorverbindungen vorliegt, wenn freie Radikale und Diradikale, deren Dimere, Oligomere, Dispiroverbindungen oder Polycyclen in neutraler Form als ein n-Dotand gegenüber einem organischen halbleitenden Matrixmaterial eingesetzt werden. Insbesondere kann durch die erfindungsgemäßen Verbindungen bei Einsatz als n-Dotand die Leitfähigkeit von Ladungstransportschichten wesentlich erhöht und/oder der Übergang der Ladungsträger zwischen den Kontakten und organischer Schicht bei Anwendungen als elektronisches Bauelement wesentlich verbessert werden. Die erfindungsgemäßen Verbindungen können auch selbst als Transportmaterialien verwendet werden. Daher können die Konzentrationsverhältnisse beim Verdampfungsprozeß zur Herstellung des organischen halbleitenden Materials zum Beispiel zwischen Radikal (oder Dimer) und konventionellen Matrixmaterialien 10:1 betragen.

Ohne durch diese Vorstellung eingeschränkt zu sein, wird davon ausgegangen, dass bei erfindungsgemäßer Verwendung der offenbarten heterocyclischen Verbindungen in einer dotierten Schicht die den jeweiligen heterocyclischen Radikalen und Diradikaine zuhörigen Kationen gebildet werden, insbesondere durch den Transfer von mindestens einem Elektron vom jeweiligen heterocyclischen Radikal und Diradikal, dessen Dimer, Oligomer, Dispiroverbindung oder Polyzyklus auf das umgebende Matrixmaterial. Ebenso werden dabei Anionen des Matrixmaterials gebildet, die auf dem Matrixmaterial beweglich sind. Auf diese Weise gewinnt das Matrixmaterial eine Leitfähigkeit, die gegenüber der Leitfähigkeit des undotierten Matrixmaterials erhöht ist. Leitfähigkeiten von undotierten Matrixmaterialien sind in der Regel < 10⁻⁸ S/cm, insbesondere häufig < 10⁻¹⁰ S/cm. Es ist dabei darauf zu achten, dass die Matrixmaterialien eine genügend hohe Reinheit aufweisen. Solche Reinheiten sind mit herkömmlichen Methoden, zum Beispiel Gradientensublimation zu erreichen. Durch Dotierung läßt sich die Leitfähigkeit solcher Matrixmaterialien auf größer 10⁻⁸ S/cm, häufig > 10⁻⁶ S/cm erhöhen. Dies gilt insbesondere für Matrixmaterialien, die ein Reduktionspotential von kleiner als -1 V vs. Fc/Fc⁺, bevorzugt kleiner -1.7 V vs. Fc/Fc⁺, insbesondere kleiner -2.1 V vs. Fc/Fc⁺ zeigen. Die Angabe Fc/Fc⁺ bezieht sich auf das Redoxpaar Ferrocen/ Ferrocenium, dass als Referenz in einer elektrochemischen Potentialbestimmung, zum Beispiel Zyklovoltammetrie eingesetzt wird.

Als Dotand wird in der vorliegenden Anmeldung einerseits ein Material verstanden, das eingemischt wird ("die Schicht wird mit dem Dotanden dotiert"). Andererseits kann der Dotand die redoxaktive Spezies sein, die mittels Ladungstranferleitfähigkeit hervorruft ("der Dotand bewirkt eine n-Dotierung"). Es wird davon ausgegangen, daß die Dimere, etc. Dotanden der ersten Art sind, während die entsprechenden Radikale Dotanden der zweiten Art darstellen.

Erfindungsgemäß wurde auch festgestellt, daß die heterocyclischen Radikale oder Diradikale sowie deren Derivate als Injektionsschicht in elektronischen Bauteilen, vorzugsweise zwischen einer Elektrode und einer Halbleiterschicht, die auch dotiert sein kann, oder auch als Blockerschicht, vorzugsweise zwischen Emitter- und Transportschicht, oder als Halbleiterschicht in elektronischen Bauelementen eingesetzt werden können. Es wurde weiter festgestellt, daß eine photo- bzw. lichtinduzierte irreversible Dotierung von organischen Halbleitern mit Hilfe der erfindungsgemäßen Verbindungen möglich ist, insbesondere die Erzeugung der genannten Radikale und Diradikale durch Spaltung ihrer Dimere bzw. Oligomere oder Dispiroverbindungen mittels elektromagnetischer Strahlung und eine nachfolgende irreversible Dotierung von n-leitenden Halbleitern.

Vorstellbar ist auch der Einsatz der erfindungsgemäßen Verbindungen als Radikalfänger bzw. Antioxidantien in der Lebensmittelchemie, der Pharmazie, bei der Brandbekämpfung oder als Schädlingsbekämpfungsmittel, insbesondere als Insektizid, Herbizid, Fungizid oder ähnliches Mittel. Auch der Einsatz als Radikalstarter für radikalische Reaktionen (vorzugsweise radikalisch induzierte Polymerisationen oder lebende radikalische Polymerisationen) ist vorstellbar. Schließlich sei erwähnt, das Triplett-Diradikale auch als magnetische Verbindung in Form von Speicher- oder Schalterstrukturen in organischen elektronischen und optoelektronischen Bauelementen verwendet werden können.

Zurückkommend auf die bevorzugte Verwendung der erfindungsgemäßen heterocyclischen Radikale, Diradikale und Derivate derselben als Dotand kann die Dotierung lichtinduziert beispielsweise nach den folgenden drei Mechanismen ablaufen:
1. Das Dimer/Oligomer/Polymer/Dispiroverbindung oder Poylcyclus selbst absorbiert elektromagnetische Strahlung geeigneter Wellenlänge und wird dadurch in die dotierenden Radikale bzw. Diradikale gespalten. Es wird ein Elektron aus dem HOMO des Radikals/Diradikals in das LUMO des Matrixmaterials übertragen.
2. Das Matrixmaterial wird durch elektromagnetische Bestrahlung angeregt, so daß in das nun einfach besetzte einstige HOMO ein Elektron aus dem HOMO des Dotanden (Dimer/Oligomer/Polymer/Dispiroverbindung/Polycyclus) transferiert wird. Der Dotand durchläuft anschließend eine irreversible Reaktion.
3. Der Dotand (Dimer/Oligomer/Polymer/Dispiroverbindung/Poylcyclus) wird photochemisch angeregt, anschließend findet ein Elektronentransfer vom einfach besetzten LUMO des Dotanden in das LUMO des Matrixmaterials statt. Der Dotand durchläuft anschließend eine irreversible Reaktion.

Es können jedoch auch verschiedene Mechanismen gleichzeitig auftreten, und schließlich kann der Elektronentransfer nach einem anderen hier nicht genannten Mechanismus, zum Beispiel durch thermische Bindungsspaltung, realisiert werden. Nach dem Abschalten der elektromagnetischen Strahlungsquelle bleibt die Leitfähigkeit jedoch vollständig oder zum Teil irreversibel bzw. dauerhaft erhalten.

Weitere Aufgaben und Vorteile der erfindungsgemäßen Verbindungen werden nun anschaulich anhand der folgenden Beispiele beschrieben, die lediglich veranschaulichend und nicht als den Umfang der Erfindung begrenzend zu betrachten sind.

Die erfindungsgemäßen Dotanden weisen eine überraschend hohe Stabilität in Bezug auf ihre Reaktivität mit der Atmosphäre auf.

### Syntheseverfahren

Die erfindungsgemäßen Radikale, Diradikale und Derivate derselben lassen sich nach bekannten Verfahren synthetisieren. Es versteht sich, daß die genannten Literaturstellen nur beispielhaft angegeben sind.

Ein vollständiger Darstellungsweg der Radikale/Diradikale soll schematisch für alle aufgeführten Verbindungen am Beispiel der Benzimidazole aufgezeigt werden.

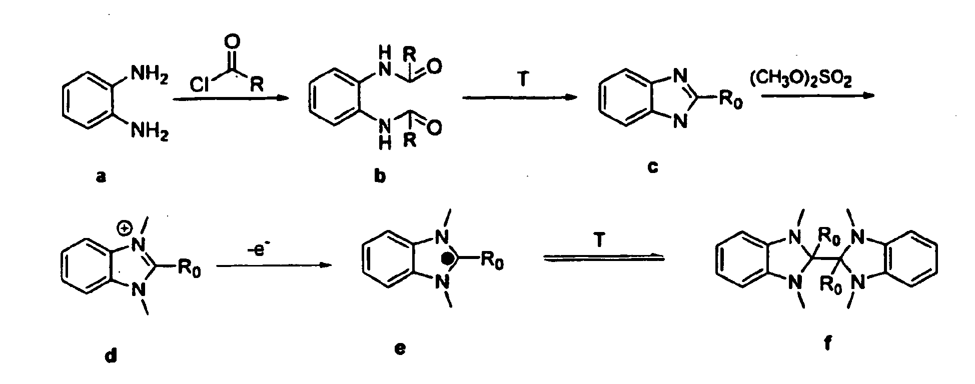

Benzimidazole **c** können unter anderem leicht aus o-Phenylendiamin **a** und entsprechenden Carbonsäurederivaten (M. R. DeLuca, S. M. Kerwin Tetrahedron 1997, 53 457-64) oder Aldehyden (M. Curini et al. Synlett 2004, 10,1832-4) synthetisiert werden. Siehe auch: M. R. Grimmett "Imidazole' and Benzimidazole Synthesis" Academic Press; Harcourt Brace & Company, Puplishers, London, San Diego, New York, Boston. o-Phenylendiamine sind käuflich oder sind beispielsweise nach der Methode von Suschitsky et al. (J. Chem. Soc. Chem. Comm. 1977, 189-90) darstellbar. Eine Darstellung von Benzthia- oder Oxazol-Derivaten kann in Analogie über die o-Mercapto- oder o-Hydroxyaniline erfolgen. Die Alkylierungen der bzw. des N-Atoms in den heterocyclischen Fünfringen c gelingt mit Dimethyl- bzw. Diethylsulfat in Gegenwart von Basen (H. Quast, E. Schmitt Chem. Ber. 1968, 101, 4012-14) oder mit Alkylhalogeniden. Die entsprechenden kationischen Produkte (Heteroarenium-Verbindungen) d können in neutraler Form z.B. als Perchlorat, Tetrafluoroborat, Halogenid, Tetraphenylborat oder Hexafluorophosphat oder mit anderen geeigneten Gegenionen isoliert werden.

Die genannten Radikale lassen sich chemisch mittels Alkalimetallen oder elektrochemisch oder photochemisch aus den entsprechenden heteroaromatischen Kationen durch Reduktion herstellen (T. Muramatsu et al. Chemistry Letters 1996, 151-2; Pragst et al. J. Electroanal. Chem. 1984, 180, 141-56, J.Heinze, H.Baumgärtel, Ber. Bunsenges. 1972 76/2 94)

In der Regel reagieren die Radikale jedoch rasch weiter zu Dimeren f, Oligomeren, Polymeren, Dispiroverbindungen i oder Polycyclen (Tricyclen) 1. Das tatsächlich Radikale als Zwischenstufen auftreten, konnte mittels ESR-Spektroskopie nachgewiesen werden.

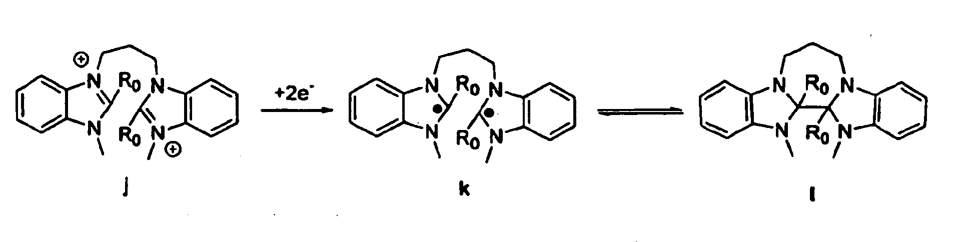

Bis-[3-methyl-2-alkyl-1,2-dihydro-benzthiazolyi-(2)]-und Bis-[3-methyl-2-aryl-1,2-dihydrobenzthiazolyl-(2)]-Verbindungen lassen sich direkt über Benzthiazoliumsalze und entsprechenden Grignard-Verbindungen darstellen /A.Kinya; S, Hiroaki; I. Naoki; Bull. Chem. Soc. Japan 1979 52/1 156-9.

Aus 2,2'-verbrückten Benzimidazolen können Dispiroverbindungen i gebildet werden.

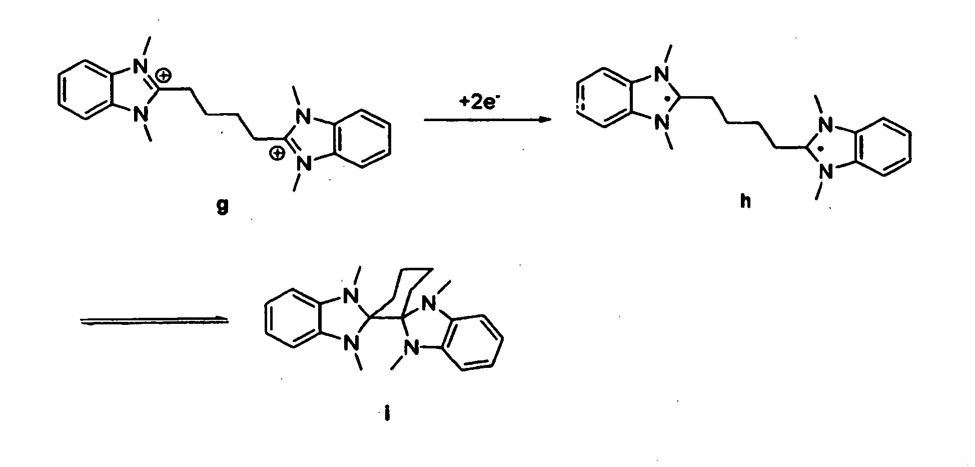

### Darstellung der N-substituierten heteroaromatischen Kationen

### Beispiel 1:

### 2-Methylmercapto-1,3-dimethylbenzimidazolium perchlorat

0,1 mol 2-Mercaptobenzimidazol in 70 ml Wasser suspendieren. 0,3 mol NaHCO₃ und 0,5 mol Dimethylsulfat zugeben und bei Raumtemperatur über Nacht rühren. In die klare Lösung wird 12ml 50% Tetrafluorborsäure zugetropft, kalt gestellt und der Niederschlag abgesaugt sowie aus 1,2-Dichlorethan umkristallisiert.
Fp.= 160-3°C

### Beispiel 2:

### 2-Piperidyl-1,3-dimethylbenzimidazolium-perchlorat

0,01 mol 2-Methylmercapto-1,3-dirnethylbenzimidazolium-perchlorat
mit 0,01 mol Piperidin 4 h am Rückfluss in 250ml Dioxan erhitzen. Feststoff absaugen und aus Ethanol umkristallisieren.
Fp. 179°C

### Beispiel 3:

### 2-Dimethylamino-benzimidazoliumchlorid

0,05mol o-Phenylendiaminiumdichlorid und 0,05mol Dichlormethylen-N,N-dimethylimmoniumchlorid in 100ml Dioxan bei Raumtemperatur 12h rühren. Anschließend 2,5-3h am Rückfluss erhitzen, absaugen und mit Ether waschen. Aus Ethanol umkristallisieren.
Fp. 293°C

### Beispiel 4:

### 1,4-Bis-1',1",3',3 "-tetramethyl-benzimidazolium-2',2"-butan

0,01 mol 1,4-Bis-benzimidazolyl-2',2"-butan in 30 ml eines Gemisches aus 50% Wasser und 50% Glykolmonomethylether suspendieren, 0,0.6 mol Natriumhydrogencarbonat und 0,05 mol Dimethylsulfat zugeben und über Nacht bei Raumtemperatur rühren. Anschließend filtrieren und mit 10 ml konz. Perchlorsäure fällen.

### Beispiel 5:

### 2,3,5,6-Tetrahydro-1H, H-3a,10b-diaza-6a-azonia-fluoranthen

0,1 mol 2-Aminobenzimidazol mit 0,2 mol 1,3-Dibrompropan und 0,3 mol KCO₃ in 250ml DMF 8h bei 120 °C erhitzen. Feststoff absaugen und das Lösungsmittel vollständig einengen und in Methanol aufnehmen sowie anschließend mit 70% Perchlorsäure versetzen. Die ausgefallenen weißen Kristalle mit Methanol, Wasser und nochmals mit Methanol waschen.
Fp.: 242°C

### Beispiel 6:

### 2-Isopropyl-1,3-dimethylimidazolium-perchlorat

0,1 mol 2-Mercaptobenzimidazol in 70 ml Wasser suspendieren. 0,3 mol NaHCO3 und 0,5 mol Dimethylsulfat zugeben und bei Raumtemperatur über Nacht rühren. In die klare Lösung wird 10ml 70% Perchlorsäure zugetropft, kalt gestellt und der Niederschlag abgesaugt sowie aus Ethanol umkristallisiert.
Fp.= 346°C

### Beispiel 7:

### Bis-(N,N',2,2'-tetramethyl-1H-benzimidazolylium)-1,3-propan-dijodid

0,02 mol NaH unter Argon in 20ml Dimethoxyethan suspendieren, unter Eiskühlung 0,02 mol 2-Methylbenzimidazol zugeben. Nach beendeter Gasentwicklung noch 60 min bei Raumtemperatur rühren und 0,01 mol 1,3-Dibrompropan zutropfen und 10 min rühren. Reaktionsgemisch bei 60 °C auf dem Wasserbad für 4,5 h erhitzen, über Nacht bei Raumtemperatur rühren lassen und auf Eis/Wasser gießen. Das ausgefallene Rohprodukt absaugen und im Vakuum trocknen. 0,005 mol dieses Zwischenproduktes in 30 ml Wasser geben, 0,015 mol NaHCO₃ und 0,015 mol Dimethylsulfat zugeben, über Nacht rühren und mit 1-2 ml konz. Jodwasserstoffsäure ausfällen.
Fp.: Zers. >306°C

### Beispiel 8:

### 1,2,3,5,6,7-Hexamethylbenzo-1,7-dihydro-benzo[1,2-d,4,5-d']diimidazolium-diperchlorat

0,013 mol 2,6-Dimethylbenzo-1,7-dihydro-benzo[1,2-d,4,5-d']diimidazol in ca. 40-50ml Wasser suspendieren und 0,078 mol NaHCO₃ und 0,064 mol Dimethylsulfat zugeben. 12 h bei Raumtemperatur rühren lassen und 4-5ml 70%-ige Perchlorsäure zutropfen. Den weißen Niederschlag absaugen, mit Ethanol, Wasser und nochmals mit Ethanol waschen.
Fp.:>350°C

### Darstellung der Radikale, Diradikale und deren Dimere bzw. Oligomere sowie Dispiroverbindungen oder Polycyclen

### Beispiel a:

### Bis-[1,3-dimethyl-2-N-piperidinyl-1,2-dihydro-benzimidazolyl-(2)]

0,01 mol 2-N-Piperidinyl-1,3-dimethylbenzimidazolium-tetrafluoroborat mit Kalium in THF am Rückfluß erhitzen, filtrieren, einengen und kühl stellen. Die ausgefallenen Kristalle absaugen und mit kaltem Acetonitril waschen.
Fp.: 195°C

### Beispiel b:

### Bis-[1,3-dimethyl-2-isopropyl-1,2-dihydro-benzimidazolyl-(2)]

1,3-Dimethyl-2-isopropylbenzimidazolium-perchlorat in 0,1 M Tetrabutylammonium-perchlorat in Acetonitril lösen und bei -2,3 V an einer Quecksilberelektrode in einer Dreikammerelektrolysezelle abscheiden. Der weiße Niederschlag wird abgesaugt, mit Acetonitril gewaschen und im Vakuum getrocknet.
Fp.:146°C

### Beispiel c:

### Bis-[1,3-dimethyl-2-N-pyrollidyl-1,2-dihydro-benzimidazolyl-(2)]

1,3-Dimethyl-2-N-pyrollidylbenzimidazolium-perchlorat in 0,1 M Tetrabutylammonium-perchlorat / DMF lösen und bei -2,3 V an einer Quecksilberelektrode in einer Dreikammerelektrolysezelle abscheiden. Der weiße Niederschlag wird abgesaugt, mit Acetonitril gewaschen und im Vakuum getrocknet.
Fp.: 120°C

### Beispiel d:

### Bis-[1,3,5,6-tetramethyl-2-isopropyl-1,2-dihydro-benzimidazolyl-(2)]

0,01 mol 1,3,5,6-Tetramethyl-2-isopropylbenzimidazoliumtetrafluoroborat mit Kalium in THF am Rückfluß erhitzen, filtrieren, einengen und kühl stellen. Die ausgefallenen Kristalle absaugen und mit kaltem Acetonitril waschen.
oder
1,3,5,6-Tetramethyl-2-isopropylbenzimidazoliumperchlorat in 0,1 M Tetrabutylammonium-perchlorat / DMF lösen und bei -2,3 V an einer Quecksilberelektrode in einer Dreikammerelektrolysezelle abscheiden. Der weiße Niederschlag wird abgesaugt, mit Acetonitril gewaschen und im Vakuum getrocknet.
Fp.: 129-30°C

### Beispiel e:

### 2-Isopropyl-1,3-dimethyl-1,3,6,7-tetrahydro-1H-5,8-dioxa-1,3-diaza-cyclopenta[b]naphthen

0,1 M Tetrabutylanunonium-perchlorat / Acetonitril lösen und bei -2,4 V an einer Quecksilberelektrode in einer Dreikammerelektrolysezelle abscheiden. Der weiße Niederschlag wird abgesaugt, mit Acetonitril gewaschen und im Vakuum getrocknet.
Fp.: 142°C

### Beispiel f

### 1,2,3,5,6,7-Hexamethylbenzo-1,7-dihydro-benzo[1,2-d,4,5-d]diimidazolyl-(2)-Oligomeres-Diradikal

0,01 mol 1,2,3,5,6,7-Hexamethylbenzo-1,7-dihydro-benzo[1,2-d,4,5-d']dümidazolium-diperchlorat in 0,1 M Tetrabutylammonium-perchlorat / DMF lösen und bei -2,3 V an einer Elektrode in einer Dreikammerelektrolysezelle abscheiden. Der weiße Niederschlag wird abgesaugt, mit Acetonitril gewaschen und im Vakuum getrocknet.
Fp.: > 250°C

### Beispiel g:

### Bis-[1,3-dimethyl-2-isopropyl-1,2,4,5,6,7-hexahydro-benzimidazolyl-(2)]

1,3-Dimethyl-2-isopropyl-4,5,6,7-tetrahydrobenzimidazolium-hexafluorophosphat in 0,1 M Tetrabutylammonium-Hexafluorophosphat in DMF lösen und bei -2,6 V an einer Quecksilberelektrode in einer Dreikammerelektrolysezelle abscheiden. Der weiße Niederschlag wird abgesaugt, mit Acetonitril gewaschen und im Vakuum getrocknet.
Fp.:127-9°C

### Beispiel h:

### Bis-[4,5-Diphenyl-2-isopropyl-1,2-dihydro-imidazolyl-(2)]

4,5-Diphenyl-2-isopropylimidazolium-hexafluorophosphat in 0,1 M Tetrabutylammonium-hexafluorophosphat in DMF lösen und bei -2,45 V an einer Quecksilberelektrode in einer Dreikammerelektrotysezelle abscheiden. Der weiße Niederschlag wird abgesaugt, mit Acetonitril gewaschen und im Vakuum getrocknet.
Fp.:160-3°C

### Beispiel i:

### Bis-[3-benzyl-2-isopropyl-1,2-dihydro-benzthiazolyl-(2)]

3-Benzyl-2-isopropylbenzthiazolium-perchlorat in 0,1 M Tetrabutylammonium-perchlorat in Acetonitril lösen und bei -2,3 V an einer Quecksilberelektrode in einer Dreikammerelektrolysezelle abscheiden. Der weiße Niederschlag wird abgesaugt, mit Acetonitril gewaschen und im Vakuum getrocknet.
Fp.:146°C

### Dotierung

### Matrixmaterialien

Als n-dotierbare Matrixmaterialen können unter anderen Quinolinatokomplexe, beispielsweise des Aluminiums oder anderer Hauptgruppenmetalle, wobei der Quinolinatoligand auch substituiert sein kann, eingesetzt werden. Insbesondere kann das Matrixmaterial Tris(8-hydroxy-quinolinato)-aluminium sein. Auch andere Aluminiumkomplexe mit O- und/oder N-Donoratomen können gegebenenfalls eingesetzt werden. Gebräuchliche Matrixmaterialien sind auch Zinkphthalocyanin (ZnPc) oder Zinktetraphenylporphyrin (ZnTPP), um nur einige Vertreter der Phthalocyanin- bzw. Porphyrinkomplexe zu nennen.

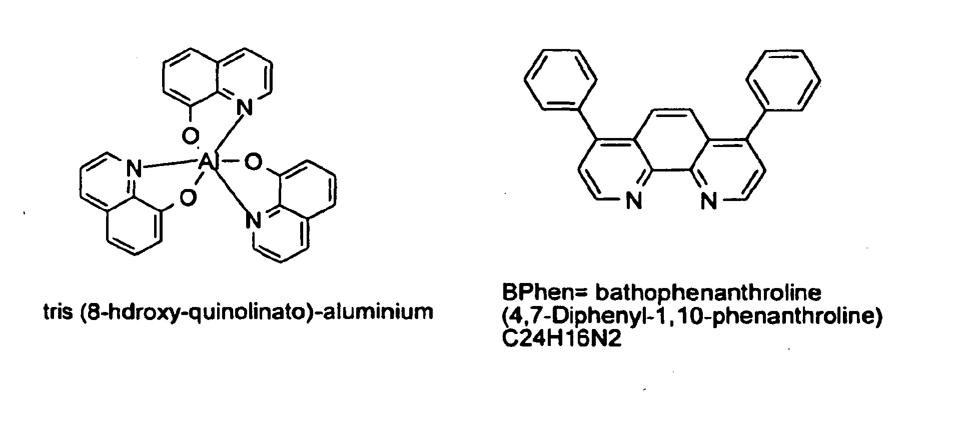

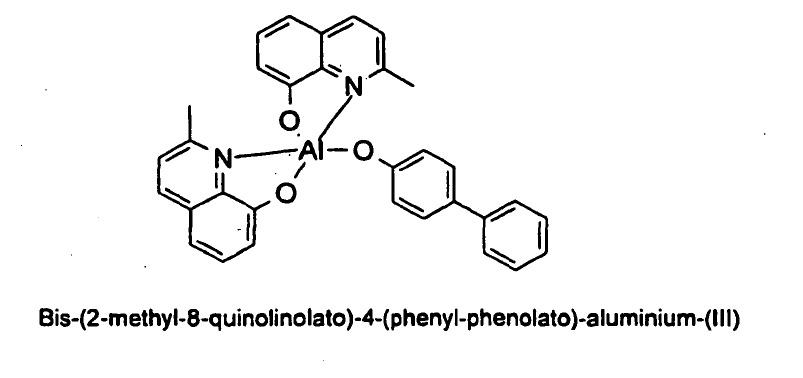

Die Quinolinatokomplexe können beispielsweise einen, zwei oder drei Quinolinatoliganden enthalten, wobei die anderen Liganden vorzugsweise mit O- und/oder N-Donoratomen an das Zentralatom komplexieren, wie beispielsweise obenstehender Al-Komplex.

Als Matrixmaterial können auch Phenanthroline eingesetzt werden, die substituiert oder unsubstituiert sein können, insbesondere Aryl-substituiert, beispielsweise Phenyl- oder Naphthyl-substituiert. Insbesondere kann Bphen als Matrixmaterial eingesetzt werden.

Als Matrixmaterial können auch Heteroaromaten wie insbesondere Triazole eingesetzt werden, gegebenenfalls auch Pyrrole, Imidazole, Triazole, Pyridine, Pyrimidine, Pyridazine Chinoxaline, Pyrazino-chinoxaline und dergleichen. Die Heteroaromaten sind vorzugsweise substituiert, insbesondere Aryl-substituiert, beispielsweise Phenyl- oder Naphthyl-substituiert. Insbesondere kann untenstehendes Triazol als Matrixmaterial eingesetzt werden. Weitere Matrixmaterialien können A.P. Kulkami et al., Chem. Mater. 16, 4556ff (2004) entnommen werden.

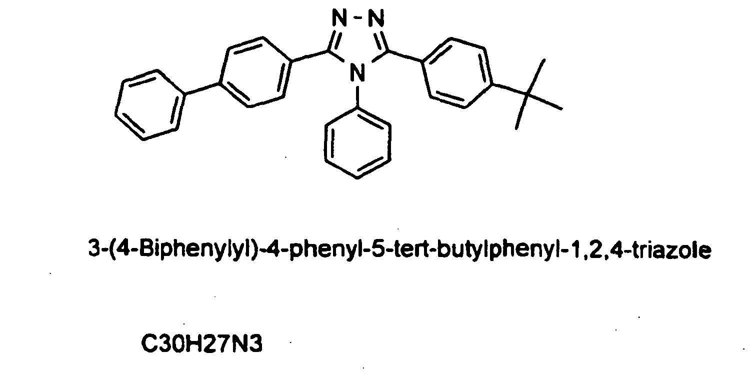

Vorzugsweise besteht das verwendete Matrixmaterial vollständig aus einem Metallphthalocyanin-Komplex, insbesondere ZnPc, einem Porphyrin-Komplex, oder einem Buckminster-Fulleren, insbesondere Fulleren C60.

Es versteht sich, dass die genannten Matrixmaterialien auch untereinander oder mit anderen Materialien gemischt im Rahmen der Erfindung einsetzbar sind. Es versteht sich, dass auch geeignete andere organische Matrixmaterialien verwendet werden können, die halbleitende Eigenschaften aufweisen.

### Dotierverfahren

Während des Dotierprozesses zerfallen die polymeren, oligomeren oder dimeren Verbindungen bzw. die Dispiroverbindungen durch Ringöffnung in die eigentlich dotierenden Radikale. Der Zerfall der Polymere, Oligomere und Dimere bzw. der Dispiroverbindungen kann auch lichtinduziert vor, während oder nach der Herstellung der Mischschicht ablaufen, daher erfolgt vorteilhaft vor, während oder nach der Mischverdampfung (Mischung aus Dotand und Matrix) eine Belichtung der Halbleiterschicht mit elektromagnetischer Strahlung, vorzugsweise mit ultraviolettem oder/und sichtbarem Licht. Ferner kann durch thermische Belastung während der Verdampfung eine Spaltung der Polymere, Oligomere und Dimere bzw. der Dispiroverbindungen in Radikale mit dotierenden Eigenschaften eintreten. Ebenso kann es für Anwendungsfälle vorteilhaft sein, die Mischschicht während oder nach der Herstellung zu erwärmen.

Die Dotierung des jeweiligen Matrixmaterials mit den erfindungsgemäßen Verbindungen kann durch eines oder eine Kombination der folgenden Verfahren hergestellt wird:
a) Mischverdampfung im Vakuum mit einer Quelle für das Matrixmaterial und einer für den Dotanden.
b) Sequentielles Deponieren des Matrixmaterials und des n-Dotanden auf einem Substrat mit anschliessender Eindiffusion des Dotanden, insbesondere durch thermische Behandlung
c) Dotierung einer Matrixschicht durch eine Lösung von n-Dotanden mit anschliessendem Verdampfen des Lösungsmittels, insbesondere durch thermische Behandlung
d) Oberflächendotierung einer Matrixmaterialschicht durch eine oberflächlich aufgebrachte Schicht von Dotanden
e) Herstellung einer Lösung von Matrixmolekülen und Dotanden und anschließende Herstellung einer Schicht aus dieser Lösung mittels konventioneller Methoden wie beispielsweise Verdampfen des Lösungsmittels oder Aufschleudern.

Die Dotierung kann gegebenenfalls auch derart erfolgen, dass der Dotand aus einer Precursor-Verbindung heraus verdampft wird, die beim Erhitzen und/oder Bestrahlen den Dotanden freisetzt. Als Precursor-Verbindung kann beispielsweise eine Carbonylverbindung, Distickstoffverbindung oder dergleichen eingesetzt werden, die bei der Freisetzung des Dotanden CO, Stickstoff oder dergleichen abspaltet, wobei auch andere geeignete Precursor einsetzbar sind, wie beispielsweise Salze, z.B. Halogenide, hydrierte Verbindungen oder dergleichen. Die Bestrahlung kann mittels elektromagnetischer Strahlung, insbesondere sichtbarem Licht, UV-Licht oder IR-Licht erfolgen, beispielsweise jeweils Laserlicht, oder auch durch andere Strahlungsarten. Durch die Bestrahlung kann im wesentlichen die zur Verdampfung notwendige Wärme bereitgestellt werden, es kann auch gezielt in bestimmte Banden der zu verdampfenden Verbindungen bzw. Precursor oder Verbindungskomplexe wie Charge-Transfer-Komplexe eingestrahlt werden, um beispielsweise durch Überführung in angeregte Zustände die Verdampfung der Verbindungen durch Dissoziation der Komplexe zu erleichtern. Der Komplex kann aber insbesondere auch ausreichend stabil sein, um unter den gegebenen Bedingungen undissoziiert zu verdampfen oder auf das Substrat aufgebracht zu werden. Es versteht sich, dass auch andere geeignete Verfahren zur Durchführung der Dotierung eingesetzt werden können.

Auf diese Weise können somit erfindungsgemäß n-dotierte Schichten von organischen Halbleitern hergestellt werden, die vielfältig einsetzbar sind.

### Anwendungsbeispiele Dotierung

Es werden ein erfindungsgemäßes Radikal oder dessen Oligomer, vorzugsweise Dimer, sowie Diradikale bzw. deren Dispiroverbindungen und Tricyclen bereitgestellt.

### Beispiel I:

Das neutrale Dimer Bis-[1,3-diethyl-2-methyl-1,2-dihydro-benzimidazolyl-(2)'] wurde zusammen mit dem Matrixmaterial Zinkphthalocyanin ZnPc verwendet. Dotierte Schichten mit einem Dotierungsverhältnis Dotand : Matrixmaterial von 1 : 20 wurden durch Mischverdampfung von Matrix und Dotand mit ZnPc als Matrixmaterial hergestellt. Die Leitfähigkeit beträgt hierbei 3x 10⁻⁴S/cm.

### Beispiel II:

Analog Beispiel I wurde eine Mischverdampfung von Bis-[1,3-dimethyl-2-isopropyl-1,2-dihydro-benzimidazolyl-(2)] und ZnPc im Verhältnis durchgeführt. Die resultierende Leitfähigkeit lag bei 10⁻³S/cm.

### Beispiel III:

Analog Beispiel I wurde eine Mischverdampfung von Bis-[1,3-dimethyl-2-isopropyl-1,2-dihydro-benzimidazolyl-(2)] und ZnTTP im Verhältnis durchgeführt. Die resultierende Leitfähigkeit lag bei 10⁻⁸S/cm.

### Beispiel IV:

Analog Beispiel 1 wurde eine Mischverdampfung von Bis-[1,3-dimethyl-2-ethyl-1,2-dihydro-benzimidazolyl-(2)] und ZnPc im Verhältnis durchgeführt. Die resultierende Leitfähigkeit lag bei 10⁻⁴ S/cm.

### Beispiel V:

Analog Beispiel I wurde eine Mischverdampfung von Bis-[1,3-dimethyl-2-N-pyrollidyl-1,2-dihydro-benzimidazolyl-(2)] und ZnTPP im Verhältnis durchgeführt. Die resultierende Leitfähigkeit lag bei 10⁻⁴S/cm.

### Beispiel VI:

Analog Beispiel I wurde eine Mischverdampfung von Bis-[1,3,5,6-tetramethyl-2-isopropyl-1,2-dihydro-benzimidazolyl-(2)] und Zinkoctaethylporphyrin ZnOEP im Verhältnis durchgeführt. Die resultierende Leitfähigkeit lag bei 5x 10⁻⁸S/cm.

### Beispiel VII:

Analog Beispiel I wurde eine Mischverdampfung von 2-Isopropyl-1,3-dimethyl-2,3,6,7-tetrahydro-1H-5,8-dioxa-1,3-diaza-cyclopenta[b]naphthen und ZnTPP im Verhältnis durchgeführt. Die resultierende Leitfähigkeit lag bei 1,8 x10⁻⁴S/cm.

### Beispiel VIII:

Analog Beispiel I wurde eine Mischverdampfung von 2-Isopropyl-1,3-dimethyl-2,3,6,7-tetrahydro-1H-5,8-dioxa-1,3-diaza-cyclopenta[b]naphthen und ZnOEP im Verhältnis durchgeführt. Die resultierende Leitfähigkeit lag bei 5x 10⁻⁸S/cm.

### Beispiel IX:

Analog Beispiel I wurde eine Mischverdampfung von 2-Isopropyl-1,3-dimethyl-2,3,6,7-tetrahydro-1H-5,8-dioxa-1,3-diaza-cyclopenta[b]naphthen und ZnPc im Verhältnis durchgeführt. Die resultierende Leitfähigkeit lag bei 2,2 x 10⁻⁸S/cm.

### Beispiel X:

Analog Beispiel I wurde eine Mischverdampfung von Bis-[1,3-dimethyl-2-isopropyl-1,2-dihydro-imidazolyl-(2)] und ZnPc im Verhältnis durchgeführt. Die resultierende Leitfähigkeit lag bei 10⁻³S/cm.

### Beispiel XI:

Analog Beispiel I wurde eine Mischverdampfung von Bis-[1,3-diethyl-2-methyl-1,2-dihydro-benzthiazolyl-(2)] und ZnPc im Verhältnis durchgeführt. Die resultierende Leitfähigkeit lag bei 3,8x 10⁻⁷S/cm.

Die in der vorstehenden Beschreibung und in den Ansprüchen offenbarten Merkmale der Erfindung können sowohl einzeln als auch in jeder beliebigen Kombination für die Verwirklichung der Erfindung in deren verschiedenen Ausführungsformen wesentlich sein.

## Patentansprüche

1. Heterocyclisches Radikal und dessen Dimer, oder Diradikal und dessen Dimer, Oligomere, Polymere, Dispiroverbindungen und Polycyclen, mit den Strukturen gemäß den folgenden Formeln: 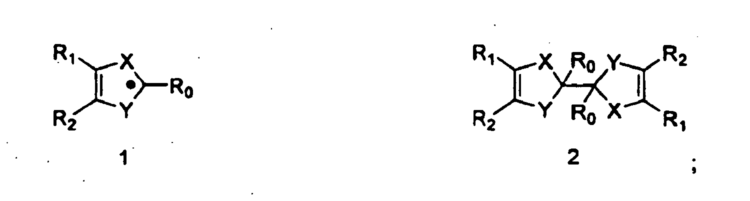 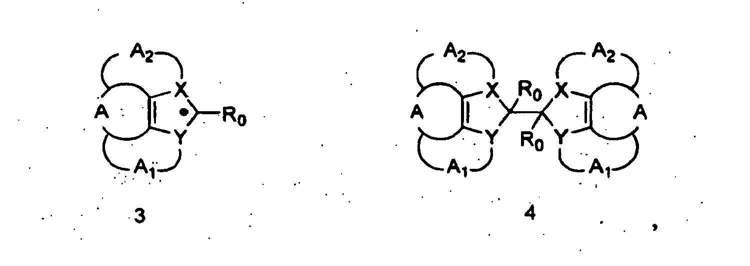 wobei die Strukturen 3 und 4 eine oder mehrere cyclische Verknüpfungen A und/oder A₁ und/oder A₂ aufweisen, wobei A, A₁ und A₂ carbocyclische, heterocyclische und/oder polycyclische Ringsysteme sein können, die substituiert oder unsubstituiert sein können; 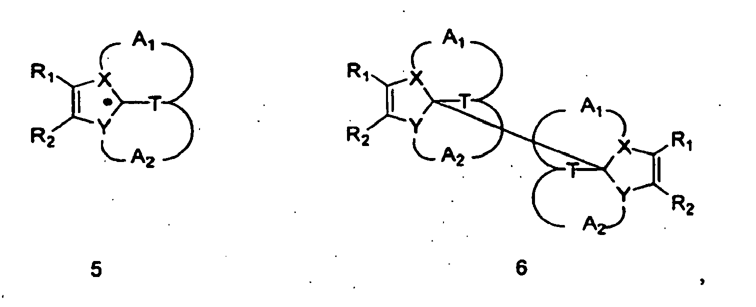 wobei A₁ und A₂ einzeln oder gemeinsam vorliegen können und A₁ und A₂ wie für Strukturen 3 und 4 definiert sind und T = CR₂₂, CR₂₂R₂₃, N, NR₂₁, O oder S ist; 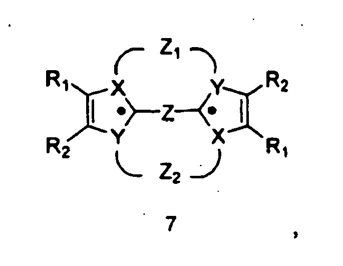 wobei Struktur 7 eine oder mehrere Brückenverbindungen Z und/oder Z₁ und/oder Z₂ aufweist, und Z, Z₁ und Z₂ unabhängig ausgewählt sein können aus Alkyl, Alkenyl, Alkinyl, Cycloalkyl, Sililyl; Alkylsililyl, Diazo, Disulfid, Heterocycloalkyl, Heterocyclyl, Piperazinyl, Dialkylether, Polyether, primärem Alkylamin, Arylamin und Polyamin, Aryl und Heteroaryl; 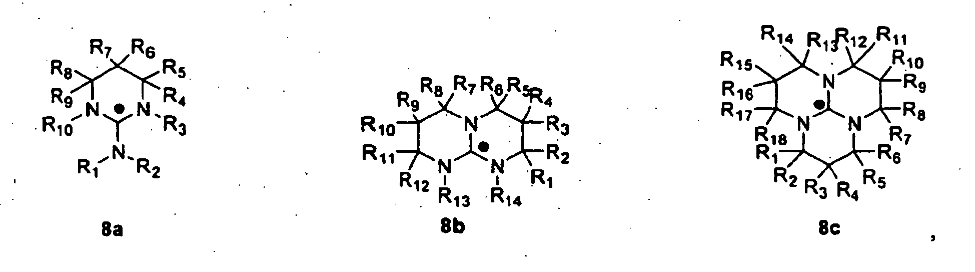 wobei in den Strukturen 8a - 8c die Ringgröße je Heterocyclus von 5-7 Atomen variieren kann;
wobei X, Y = O, S, N, NR₂₁, P, oder PR₂₁ ist; R₀₋₁₉, R₂₁, R₂₂ und R₂₃ unabhängig ausgewählt sind aus, substituiert oder unsubstituiert, Aryl, Heteroaryl, Heterocyclyl, Diarylamin, Diheteroarylamin, Dialkylamin, Heteroarylalkylamin, Arylalkylamin, H, F Cycloalkyl, Halogencycloalkyl, Heterocycloalkyl, Alkyl, Alkenyl, Alkinyl, Trialkylsilyl, Triarylsilyl, Halogen, Styryl, Alkoxy, Aryloxy, Thioalkoxy, Thioaryloxy, Sililyl und Trialkylsilylalkinyl, oder R₀₋₁₉, R₂₁, R₂₂ und R₂₃, allein oder in Kombination, Teil eines Ringsystems, (hetero)aliphatisch oder (hetero)aromatisch, sind ;
mit der Maßgabe, daß die folgenden Strukturen ausgeschlossen sind:
Formeln 1, 2, 3 und 4, in denen R₀ Wasserstoff ist;
Formel 4, in der Y=NR₂₁ ist und R₂₁ Wasserstoff ist;
Struktur 3a 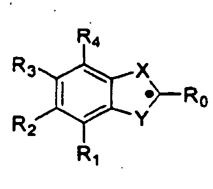 mit R₁- R₄ = H, X = NCH₃, Y = NCH₃ und R₀ = Methyl;
Struktur 3a 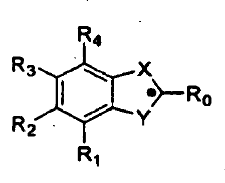 mit X = NCH₃, Y = S und R₀ = Methyl, Ethyl, Butyl, Phenyl, 4-Tolyl, 4-Anisyl, 4-Chlorphenyl;
Struktur 1 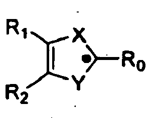 mit X = N₂₁, Y = NR₂₂ und R₁, ₂, ₂₁, ₂₂ = Phenyl, 4-Tolyl und/oder 4-Anisyl, R₀ = Phenyl, 4-Tolyl oder 4-Anisyl.

2. Heterocyclisches Radikal und dessen Dimer, oder Diradikal und dessen Dimer, Oligomere, Polymere, Dispiroverbindungen und Polycyclen nach Anspruch 1, **dadurch gekennzeichnet, daß** A₁ A₁ und A₂ ausgewählt sind aus substituierten oder nicht-substituierten, aromatischen und heteroaromatischen Ringsystemen, vorzugsweise Benzo-, Naphtho-, Thiophen-, Furan-, Thiazol-, Imidazol-, Oxazol-, Thiadiazol-; Pyrazin-, Thiopyran-, Dithiin-, Phthalsäureimid- und Dithiazol-Resten, wobei eine oder mehrere Substituenten vorliegen können, die ausgewählt sind aus R₀ - R₁₉, R₂₁, R₂₂ und R₂₃.

3. Heterocyclisches Radikal und dessen Dimer, oder Diradikal und dessen Dimer, Oligomere, Polymere, Dispiroverbindungen und Polycyclen nach Anspruch 1 oder 2, **dadurch gekennzeichnet, daß** Z, Z₁ und Z₂ ausgewählt sind aus Piperazinyl und Alkyl bzw. Cycloalkyl

4. Heterocyclisches Radikal und dessen Dimer, oder Diradikal und dessen Dimer, Oligomere, Polymere, Dispiroverbindungen und Polycyclen nach einem der vorangehenden Ansprüche, **gekennzeichnet durch** eine der folgenden Strukturen: 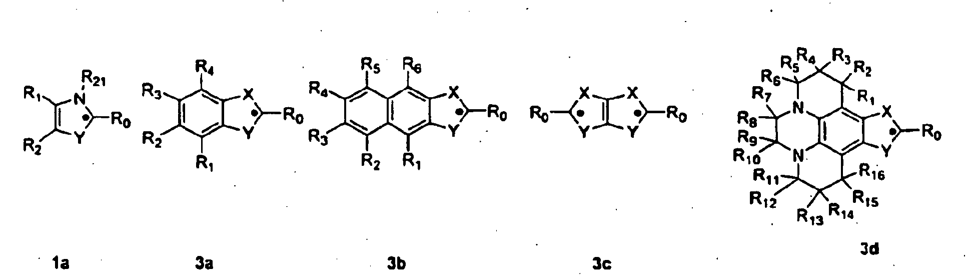 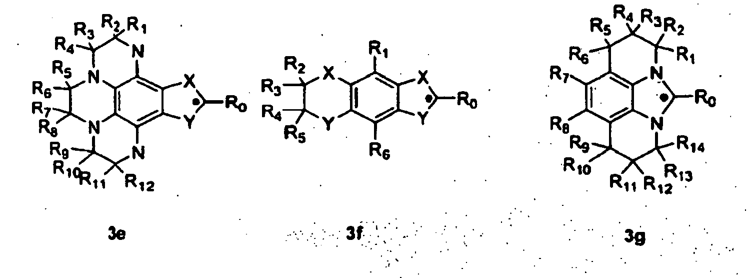 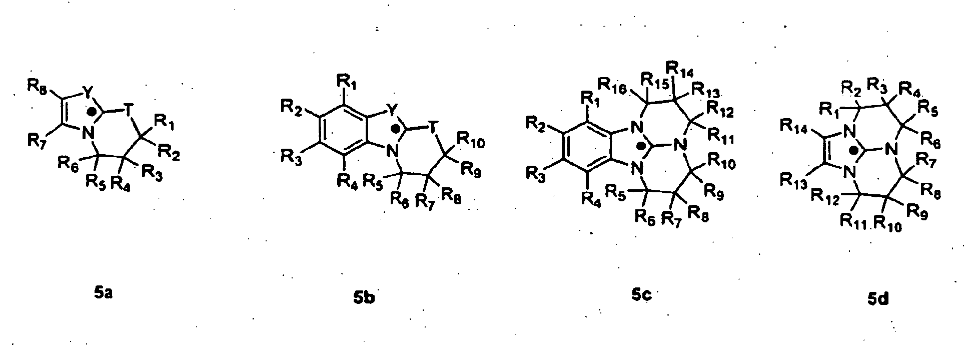 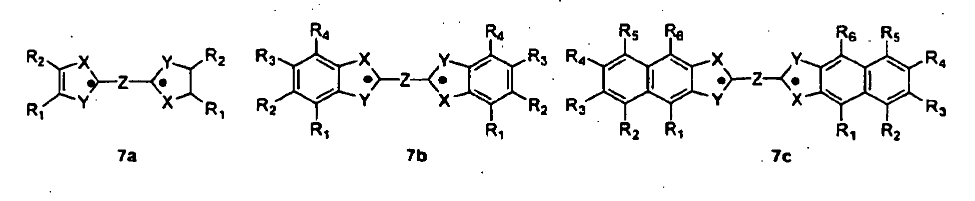 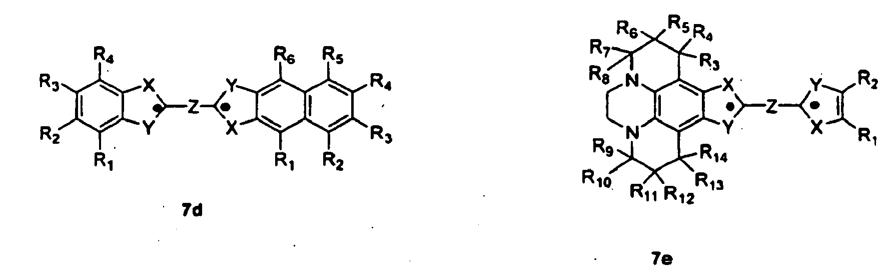 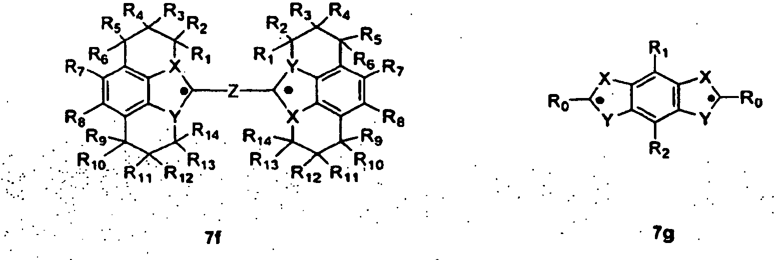 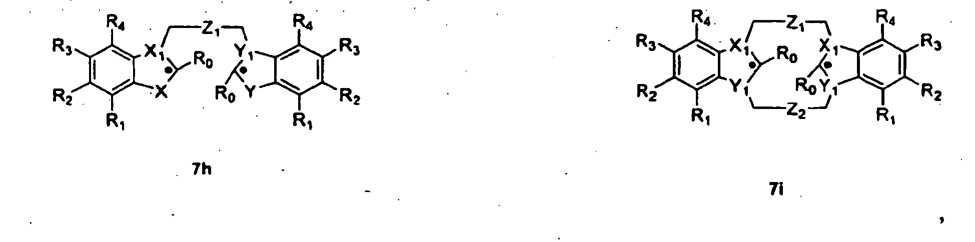 wobei X₁, Y₁ = N oder P ist.

5. Heterocyclisches Radikal und dessen Dimer, oder Diradikal und dessen Dimer, Oligomere, Polymere, Dispiroverbindungen und Polycyclen nach einem der vorangehenden Ansprüche, **dadurch gekennzeichnet, daß** R₀₋₁₉, R₂₁, R₂₂ und R₂₃ ausgewählt sind aus, substituiert oder nicht substituiert Phenyl, Biphenyl, Naphthyl, Anthranyl, Thienyl, Imidazolyl, Pyrrolyl, Thiazolyl, Oxazolyl, Thiadiazolyl, Piperidyl, Pyrrolidyl, Morpholyl und Thiomorpholyl.

6. Heterocyclisches Radikal und dessen Dimer, oder Diradikal und dessen Dimer, Oligomere, Polymere, Dispiroverbindungen und Polycyclen nach einem der vorangehenden Ansprüche, **dadurch gekennzeichnet, daß** R₀₋₁₉, R₂₁, R₂₂ und R₂₃ ausgewählt sind aus, Alkyl, Cykloalkyl, Dialkylamin, Diarylamin, Alkoxy, Aryloxy, Thioaryl, Thioalkoxy, Perfluoralkyl.

7. Heterocylisches Radikal und dessen Dimer, oder Diradikal und dessen Dimer, Oligomere, Polymere, Dispiroverbindungen und Polycyclen nach einem der vorangehenden Ansprüchen, **dadurch gekennzeichnet, daß** der Polycyclus ein Tricyclus ist.

8. Verwendung von heterocyclischem Radikal und dessen Dimer, oder Diradikal und dessen. Dimere, Oligomeren, Polymeren, Dispiroverbindungen und Polycyclen nach einem der Ansprüche 1 bis 6 als Dotand zur Dotierung eines organischen halbleitenden Matrixmaterials, als Blockerschicht, als Ladungsinjektionsschicht, als Elektrodenmaterial, als Speichermaterial oder als Halbleiterschicht selbst in elektronischen und optoelektronischen Bauelementen.

9. Verwendung nach Anspruch 8, **dadurch gekennzeichnet, daß** die heterocyclische Verbindung als Kation vorliegt.

10. Organisches halbleitendes Material enthaltend zumindest eine organische Matrixverbindung und einen Dotanden, **dadurch gekennzeichnet, daß** der Dotand zumindest eine Verbindung gemäß den Ansprüchen 1 bis 7 ist.

11. Organisches halbleitendes Material nach Anspruch 10, **dadurch gekennzeichnet, daß** das molare Dotierungsverhältnis von Dotand zu Matrixmolekül bzw. das Dotierungsverhältnis von Dotand zu monomeren Einheiten eines polymeren Matrixmoleküls zwischen 1:1 und 1:100.000 beträgt.

12. Organisches halbleitendes Material nach Anspruch 10 oder 11, **dadurch gekennzeichnet, daß** der Dotand als Kation vorliegt.

13. Verfahren zur Herstellung eines organischen halbleitenden Materials enthaltend ein organisches Matrixmolekül und einen Dotanden, **dadurch gekennzeichnet, daß** als Dotand zumindest eine Verbindung nach einem der Ansprüche 1 bis 7 verwendet wird.

14. Elektronisches oder optoelektronisches Bauelement mit einem elektronisch funktionell wirksamen Bereich, **dadurch gekennzeichnet, daß** der elektronisch wirksame Bereich zumindest eine der Verbindungen der Ansprüche 1 bis 7 umfaßt.

15. Elektronisches oder optoelektronisches Bauelement nach Anspruch 14, **dadurch gekennzeichnet, daß** der elektronisch wirksame Bereich ein organisches halbleitendes Matrixmaterial aufweist, welches mit zumindest einem Dotanden zur Veränderung der elektronischen Eigenschaften des halbleitenden Matrixmaterials unter Verwendung zumindest einer Verbindung der Ansprüche 1 bis 7 dotiert ist.

16. Elektronisches oder optoelektronisches Bauelement nach Anspruch 14 oder 15 in Form einer organischen Licht-emittierenden Diode, einer photovoltaischen Zelle, einer organischen Solarzelle, einer organischen Diode, eines organischen Feldeffekttransistors oder eines photoinitiierten sowie magnetischen Speichers.

17. Elektronisches oder optoelektronisches Bauelement nach einem der Ansprüche 14 bis 16, **dadurch gekennzeichnet, daß** die Verbindung der Ansprüche 1 bis 7 als Kation vorliegt.

## Claims

1. Heterocyclic radical and the dimer thereof, or diradical and the dimer, oligomers, polymers, dispiro compounds and polycycles thereof having the structures according to the following formulae: 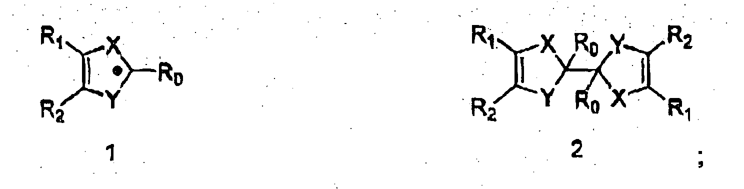 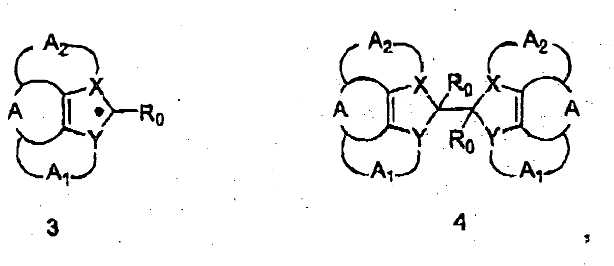 wherein structures 3 and 4 have one or more cyclic linkages A and/or A₁ and/ or A₂, wherein A, A₁ and A₂ may be carbocyclic, heterocyclic and/or polycyclic ring systems, which may be substituted or unsubstituted; 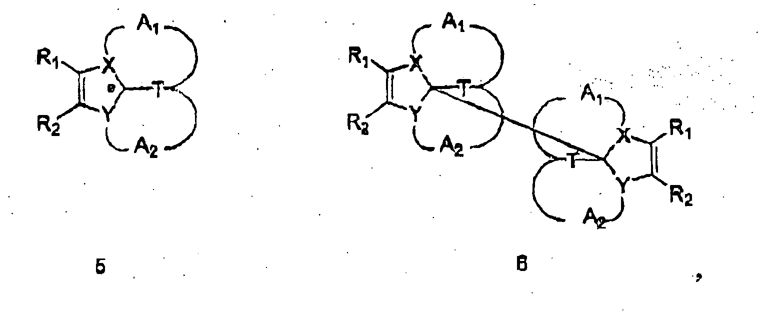 wherein A₁ and A₂ may be present individually or together and A₁ and A₂ are as defined for structures 3 and 4 and T=CR₂₂, CR₂₂R₂₃, N, NR₂₁, O or S; 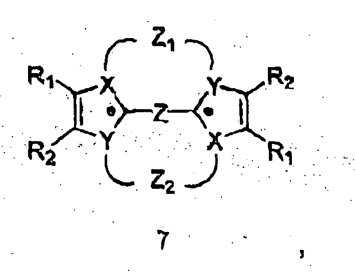 wherein structure 7 has one or more bridge bonds Z and/or Z₁ and/or Z₂, and Z, Z₁ and Z₂ may independently be selected from alkyl, alkenyl, alkynyl, cycloalkyl, sililyl; alkylsililyl, diazo, disulphide, heterocycloalkyl, heterocyclyl, piperazinyl, dialkyl ether, polyether, primary alkylamine, arylamine and polyamine, aryl and heteroaryl; 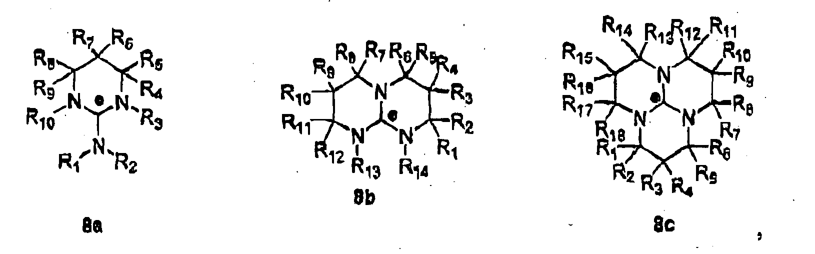 wherein in structures 8a-8c the ring size of each heterocycle may vary from 5-7 atoms;
wherein X, Y = O, S, N, NR₂₁, P or PR₂₁; R₀₋₁₉, R₂₁, R₂₂ and R₂₃ are independently selected from, substituted or unsubstituted, aryl, heteroaryl, heterocyclyl, diarylamine, diheteroarylamine, dialkylamine, heteroarylalkylamine, arylalkylamine, H, F, cycloalkyl, halocycloalkyl, heterocycloalkyl, alkyl, alkenyl, alkynyl, trialkylsilyl, triarylsilyl, halogen, styryl, alkoxy, aryloxy, thioalkoxy, thioaryloxy, sililyl and trialkylsilylalkynyl, or R₀₋₁₉, R₂₁, R₂₂ and R₂₃, alone or in combination, form part of a (hetero) aliphatic or (hetero)aromatic ring system;
with the proviso that the following structures are excluded:
formulae 1, 2, 3 and 4 in which R₀ is hydrogen;
formula 4 in which Y = NR₂₁ and R₂₁ is hydrogen; 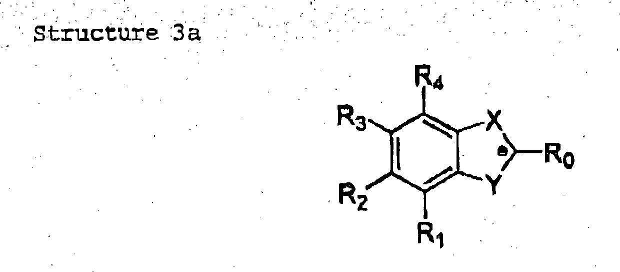
where R₁-R₄ = H, X = NCH₃, Y = NCH₃ and R₀=methyl ; 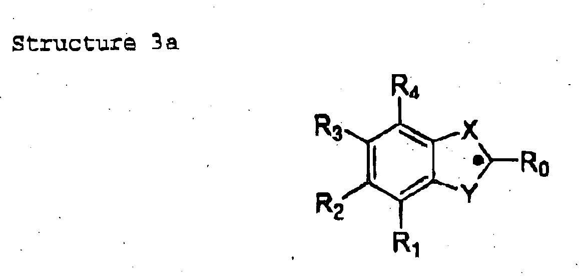 where X = NCH₃, Y = S and R₀=methyl, ethyl, butyl, phenyl, 4-tolyl, 4-anisyl, 4-chlorophenyl; 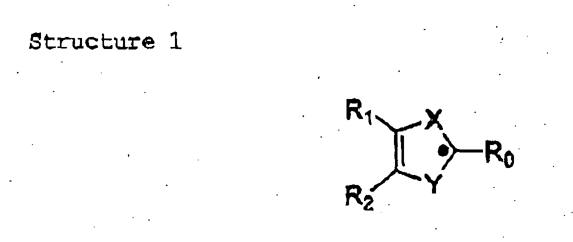 where X = N₂₁, Y = NR₂₂ and R₁, ₂, ₂₁, ₂₂ = phenyl, 4-tolyl and/or 4-anisyl, R₀=phenyl, 4-tolyl or 4-anisyl.

2. Heterocyclic radical and the dimer thereof, or diradical and the dimer, oligomers, polymers, dispiro compounds and polycycles thereof according to Claim 1, **characterized in that** A, A₁ and A₂ are selected from substituted or unsubstituted, aromatic and heteroaromatic ring systems, preferably benzo, naphtho, thiophene, furan, thiazole, imidazole, axazole; thiadiazole, pyrazine, thiopyran, dithiine, phthalic acid imide and dithiazole radicals, wherein one or mora substituents may be present, which are selected from R₀-R₁₉, R₂₁ , R₂₂ and R₂₃.

3. Heterocyclic radical and the dimer thereof, or diradical and the dimer, oligomers, polymers, dispiro compounds and polycycles thereof according to Claim 1 or 2, **characterized in that** Z, Z₁, and Z₂ are selected from piperazinyl and alkyl or cycloalkyl.

4. Heterocyclic radical and the dimer thereof, or diradical and the dimer, oligomers, polymers, dispiro compounds and polycycles thereof according to any one of the preceding claims, **characterized by** one of the following structures: 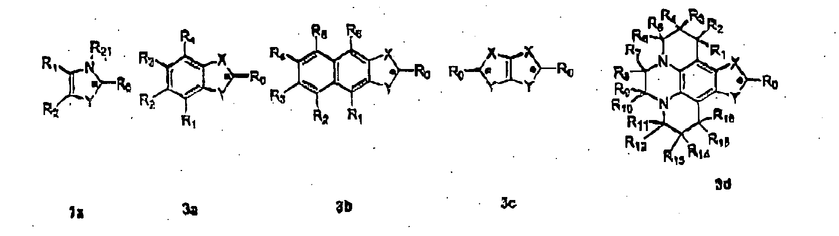 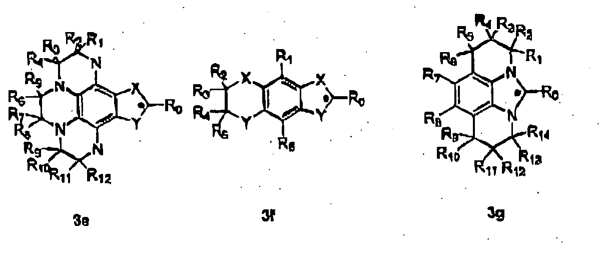 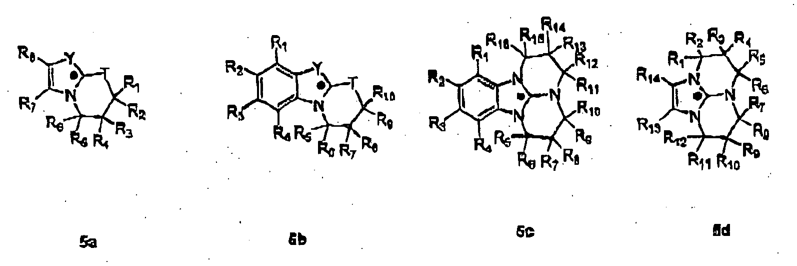 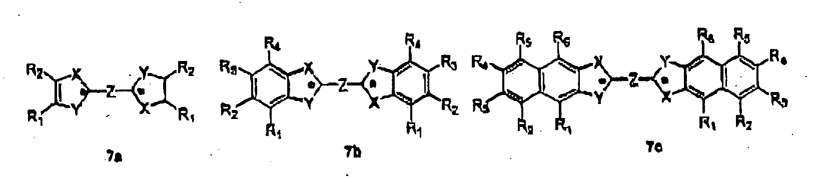 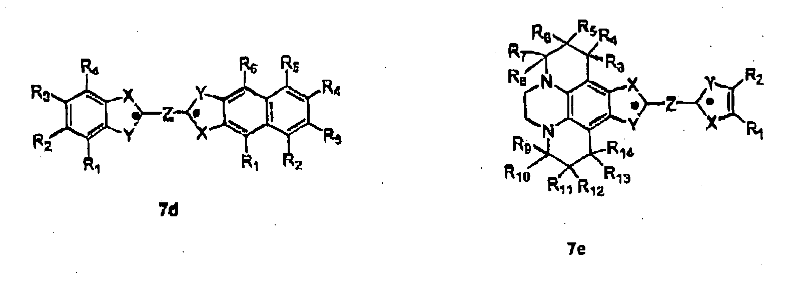 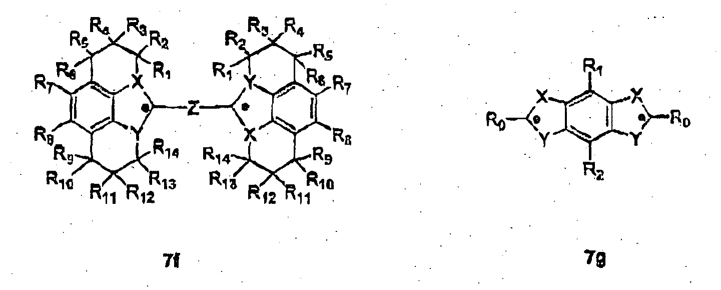 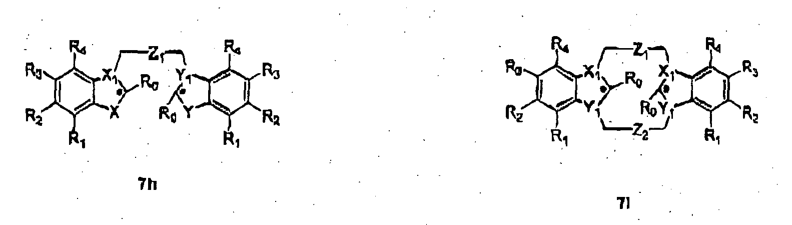 wherein X₁, Y₁ = N or P.

5. Heterocyclic radical and the dimer thereof, or diradical and the dimer, oligomers, polymers, dispiro compounds and polycycles thereof according to any one of the preceding claims, **characterized in that** R₀₋₁₉, R₂₁, R₂₂ and R₂₃ are selected from substituted or unsubstituted phenyl, biphenyl, naphthyl,- anthranyl, thienyl, imidazolyl, pyrrolyl, thiazolyl, oxazolyl, thiadiazolyl, piperidyl, pyrrolidyl, morpholyl and thiomorpholyl.

6. Heterocyclic radical and the dimer thereof, or diradical and the dimer, oligomers, polymers, dispiro compounds and polycycles thereof according to any one of the preceding claims, **characterized in that** R₀₋₁₉, R₂₁, R₃₂ and R₂₃ are selected from alkyl, cycloalkyl, dialkylamine, diarylamine, alkoxy, aryloxy, thioaryl, thioalkoxy, perfluoroalkyl.

7. Heterocylic radical and the dimer thereof, or diradical and the dimer, oligomers, polymers, dispiro compounds and polycycles thereof according to any one of the preceding claims, **characterized in that** the polycycle is a tricycle.

8. Use of the heterocyclic radical and the dimer thereof, or diradical and the dimers, oligomers, polymers, dispiro compounds and polycycles thereof according to any one of Claims 1 to 6 as a dopant for doping an organic semiconductive matrix material, as a blocking layer, as a charge injection layer, as an electrode material, as a memory material or as a semiconductor layer itself in electronic and optoelectronic components.

9. Use according to Claim 8, **characterized in that** the heterocyclic compound is present as a cation.

10. Organic semiconductive material containing at least one organic matrix compound and a dopant, **characterized in that** the dopant is at least one compound according to Claims 1 to 7.

11. organic semiconductive material, according to Claim 10, **characterized in that** the molar doping ratio of dopant to matrix molecule or the doping ratio of dopant to monomeric units of a polymeric matrix molecule is between 1:1 and 1:100,000.

12. Organic semiconductive material according to Claim 10 or 11, **characterized in that** the dopant is present as a cation.

13. Method for producing an organic semiconductive material containing an organic matrix molecule and a dopant, **characterized in that** at least one compound according to any one of Claims 1 to 7 is used as dopant .

14. Electronic or optoelectronic component having an electronically functionally active area, **characterized in that** the electronically active area comprises at least one of the compounds according to Claims 1 to 7.

15. Electronic or optoelectronic component according to Claim 14, **characterized in that** the electronically active area comprises an organic semiconductive matrix material which is doped with at least one dopant to modify the electronic properties of the semiconductive matrix material, using at least one compound according to Claims 1 to 7.

16. Electronic or optoelectronic component according to Claim 14 or 15 in the form of an organic light-emitting diode, a photovoltaic cell, an organic solar cell, an organic diode, an organic field effect transistor or a photoinitiated and magnetic memory.

17. Electronic or optoelectronic component according to any one of Claims 14 to 16, **characterized in that** the compound according to Claims 1 to 7 is present as a cation.

## Revendications

1. Radical hétérocyclique et son dimère, ou bien diradical et son dimère, ses oligomères, ses polymères, ses composés dispiro et polycycliques, présentant les structures qui correspondent aux formules suivantes : 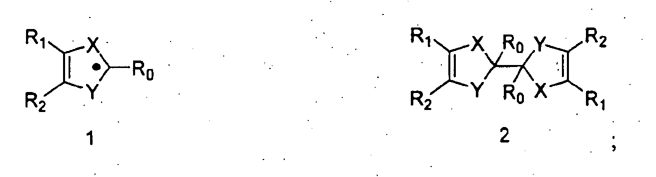 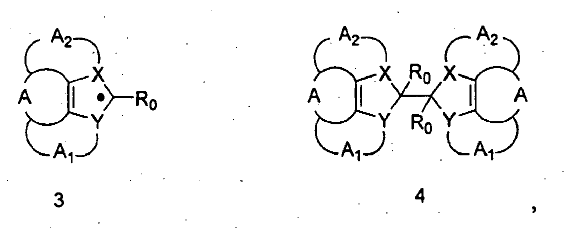 dans lequel les structures 3 et 4 présentent une ou plusieurs liaisons cycliques A et/ou A₁ et/ou A₂, où A, A₁ et A₂ peuvent être des systèmes de noyaux carbocycliques, hétérocycliques et/ou polycycliques pouvant être substitués ou non substitués ; 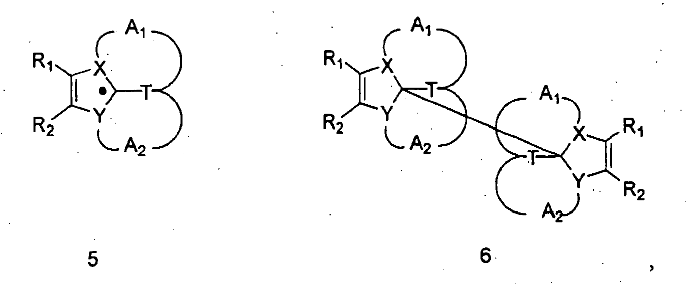 où A₁ et A₂ peuvent être présents isolément ou ensemble et A₁ et A₂ ont les mêmes définitions que dans le cas des structures 3 et 4, et T est CR₂₂, CR₂₂R₂₃, N, NR₂₁, O ou S ; 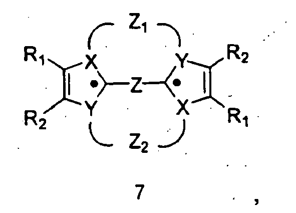 où la structure 7 présente un ou plusieurs composés de pontage Z et/ou Z₁ et/ou Z₂, et Z, Z₁ et Z₂ peuvent être choisis indépendamment les uns des autres parmi les groupes alkyle, alcényle, alcynyle, cycloalkyle, sililyle, alkyl-sililyle, diazo, disulfure, hétérocycloalkyle, hétérocyclyle, pipérazinyle, dialkyléther, polyéther, alkylamine primaire, arylamine et polyamine, aryle et hétéroaryle ; 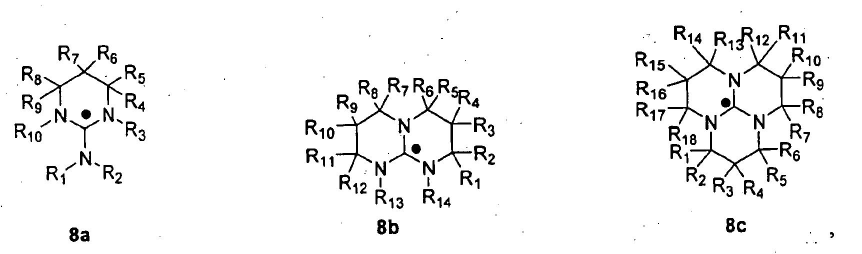 la taille des noyaux hétérocycliques des structures 8a à 8c pouvant varier de 5 à 7 atomes ;
X et Y sont O, S, N, NR₂₁, P ou PR₂₁ ; R₀ à R₁₉, R₂₁, R₂₂ et R₂₃ sont choisis indépendamment parmi des groupes aryle, hétéroaryle, hétérocyclyle, diarylamine, dihétéroarylamine, dialkylamine, hétéroarylalkylamine, arylalkylamine, H, F, cycloalkyle, halocycloalkyle, hétérocycloalkyle, alkyle, alcényle, alcynyle, trialkylsilyle, triarylsilyle, halogène, styryle, alcoxy, aryloxy, thioalcoxy, thioaryloxy, sililyle et trialkylsilylalcynyle substitués ou non substitués, ou bien R₀ à R₁₉, R₂₁, R₂₂ et R₂₃, seuls ou en combinaison, font partie d'un système de noyaux (hétéro)aliphatiques ou (hétéro)aromatiques ;
à l'exclusion des formules suivantes:
Formules 1, 2, 3 et 4 dans lesquelles R₀ est l'hydrogène
Formule 4 dans laquelle Y est NR₂₁ et R₂₁ est l'hydrogène;
Structure 3a 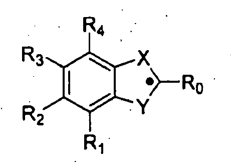 R₁ à R₄ = H, X = NCH₃, Y = NCH₃ et R₀ = méthyle ;
Structure 3a 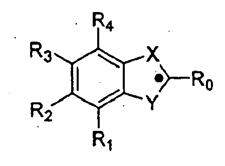 X = NCH₃, Y - S et R₀ = méthyle, éthyle, butyle, phényle, 4-tolyle, 4-anisyle, 4-chlorophényle ;
Structure 1 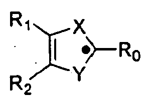 avec X = N₂₁, Y = NR₂₂ et R_{1, 2, 21, 22} = phényle, 4-tolyle et/ou 4-anisyle, R₀ =phényle, 4-tolyle ou 4-anisyle.

2. Radical hétérocyclique et son dimère, ou bien diradical et son dimère, ses oligomères, ses polymères, ses composés dispiro et polycycliques selon la revendication 1, **caractérisé en ce que** A, A₁ et A₂ sont choi-sis parmi des systèmes de noyaux aromatiques et hétéro-aromatiques, substitués ou non substitués, de préférence des restes benzo, naphto, thiophène, furane, thiazole, imidazole, oxazole, thiadiazole, pyrazine, thiopyrane, dithiine, phtal-imide et dithiazole, un ou plusieurs substituants choisis parmi R₀ à R₁₉, R₂₁, R₂₂ et R₂₃ pouvant être présents.

3. Radical hétérocyclique et son dimère, ou bien diradical et son dimère, ses oligomères, ses polymères, ses composés dispiro et polycycliques selon la revendication 1 ou 2, **caractérisé en ce que** Z, Z₁ et Z₂ sont choisis parmi les groupes pipérazinyle et alkyle ou cycloalkyle.

4. Radical hétérocyclique et son dimère, ou bien diradical et son dimère, ses oligomères, ses polymères, ses composés dispiro et polycycliques selon l'une des revendications précédentes, **caractérisé par** une des structures suivantes: 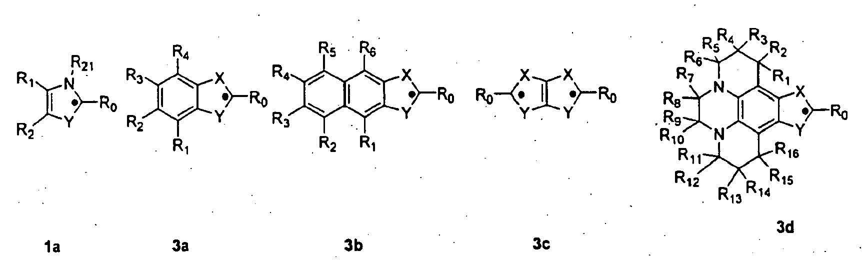 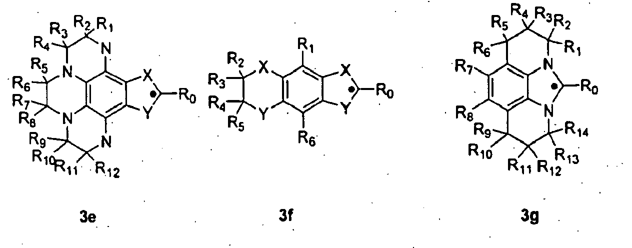 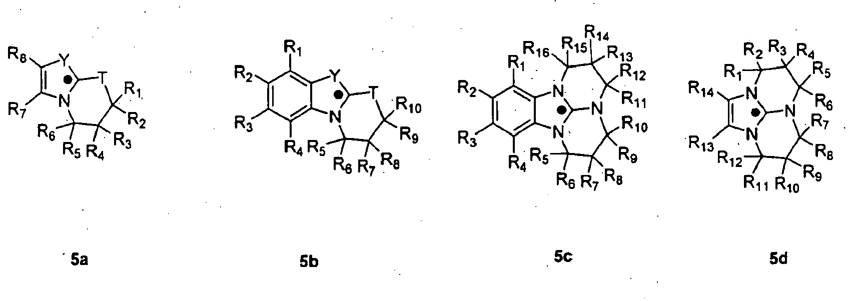 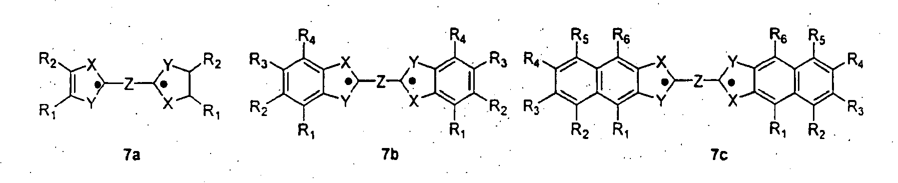 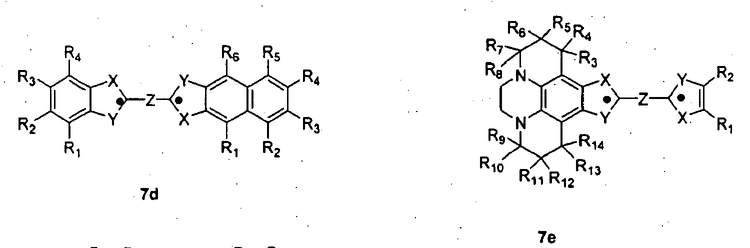 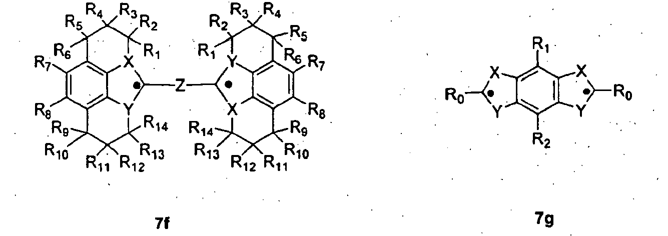 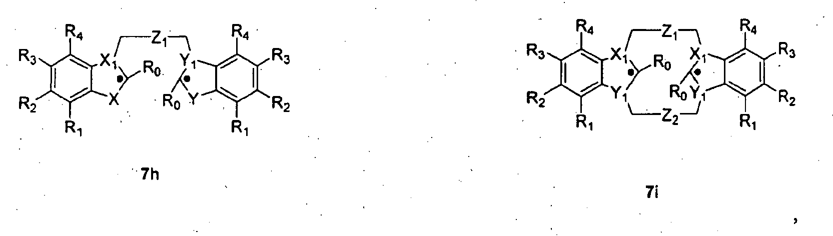 dans lesquelles X₁ et Y₁ sont N ou P.

5. Radical hétérocyclique et son dimère, ou bien diradical et son dimère, ses oligomères, ses polymères, ses composés dispiro et polycycliques selon l'une des revendications précédentes, **caractérisé en ce que** R₀ à R₁₉, R₂₁, R₂₂ et R₂₃ sont choisis parmi des groupes phényle, biphényle, naphtyle, anthranyle, thiényle, imidazolyle, pyrrolyle, thiazolyle, oxazolyle, thiadiazolyle, pipéridyle, pyrrolidyle, morpholyle et thiomorpholyle substitués ou non substitués.

6. Radical hétérocyclique et son dimère, ou bien diradical et son dimère, ses oligomères, ses polymères, ses composés dispiro et polycycliques selon l'une des revendications précédentes, **caractérisé en ce que** R₀ à R₁₉, R₂₁, R₂₂ et R₂₃ sont choisis parmi des groupes alkyle, cycloalkyle, dialkylamine, diarylamine, alcoxy, aryloxy, thioaryle, thioalcoxy, perfluoralkyle.

7. Radical hétérocyclique et son dimère, ou bien diradical et son dimère, ses oligomères, ses polymères, ses composés dispiro et polycycliques selon l'une des revendications précédentes, **caractérisé en ce que** le composé polycyclique est un système tricyclique.

8. Utilisation du radical hétérocyclique et de son dimère, ou du diradical et de ses dimères, oligomères, polymères, composés dispiro et polycycliques selon l'une des revendications 1 à 6 comme agent dopant pour doper un matériau de matrice semi-conducteur organique, comme couche de blocage, comme couche d'injection de charges, comme matériau d'électrodes, comme matériau de mémoire ou comme couche semi-conductrice proprement dite dans des composants électroniques et optoélectroniques.

9. Utilisation selon la revendication 8, **caractérisée en ce que** le composé hétérocyclique est présent sous la forme d'un cation.

10. Matériau semi-conducteur organique contenant au moins un composé de matrice organique et un agent dopant, **caractérisé en ce que** l'agent dopant est au moins un composé selon les revendications 1 à 7.

11. Matériau semi-conducteur organique selon la revendication 10, **caractérisé en ce que** le rapport molaire de l'agent dopant sur la molécule de matrice ou le rapport molaire de l'agent dopant sur les motifs monomères d'une molécule de matrice polymère se situe dans la plage de 1:1 à 1:100 000.

12. Matériau semi-conducteur organique selon la revendication 10 ou la revendication 11, **caractérisé en ce que** l'agent dopant est présent sous la forme d'un cation.

13. Procédé de fabrication d'un matériau semi-conducteur organique contenant une molécule de matrice organique et un agent dopant, **caractérisé en ce que** l'agent dopant utilisé est au moins un composé selon l'une des revendications 1 à 7.

14. Composant électronique ou optoélectronique ayant une zone fonctionnellement active du point de vue électronique, **caractérisé en ce que** la zone active du point de vue électronique comprend au moins un des composés selon les revendications 1 à 7.

15. Composant électronique ou optoélectronique selon la revendication 14,**caractérisé en ce que** la zone active du point de vue électronique présente un matériau de matrice semi-conducteur organique qui est dopé avec au moins un agent dopant afin de modifier les propriétés électroniques du matériau de matrice semi-conducteur en utilisant au moins un composé selon les revendications 1 à 7.

16. Composant électronique ou optoélectronique selon la revendication 14 ou la revendication 15, sous la forme d'une diode électroluminescente organique, d'une cellule photovoltaïque, d'une cellule solaire organique, d'une diode organique, d'un transistor à effet de champ organique ou d'une mémoire photo-excitée ainsi que magnétique.

17. Composant électronique ou optoélectronique selon l'une des revendications 14 à 16, **caractérisé en ce que** le composé selon les revendications 1 à 7 est présent sous la forme d'un cation.
